# EUROPEAN PATENT SPECIFICATION

(11) **EP 0 971 660 B1**
(45) Date of publication and mention of the grant of the patent: **01.09.2004**
(21) Application number: 95933229.7
(22) Date of filing: 20.09.1995
(51) Int. Cl.: A61F 11/04, A61D 7/00, A61M 23/00, A61N 1/375

(54) **BIORESORBABLE POLYMER USE IN COCHLEAR AND OTHER IMPLANTS**
BIORESORBIEBARES POLYMER FÜR COCHLEAR UND ANDERE IMPLANTATE
UTILISATION DE POLYMERES BIO-RESORBABLES DANS DES IMPLANTS COCHLEAIRES ET AUTRES

(43) Date of publication of application: 19.01.2000
(73) Proprietor: Cochlear Limited, Lane Cove, NSW 2066 (AU)
(72) Inventor: PARKER, John, Lane Cove, NSW 2066 (AU); TREABA, Claudiu, Wollstonecraft, NSW 2065 (AU)
(74) Representative: Molyneaux, Martyn William
(86) International application number: PCT/AU1995/000622
(87) International publication number: WO 1997/010784

(56) References cited:
- EP-A- 0 002 068
- EP-A- 0 350 188
- WO-A-83/00011
- WO-A-93/06698
- AU-A- 5 251 079
- AU-A- 5 962 380
- AU-A- 7 100 281
- NL-A- 6 709 780

## Description

This invention pertains to implantable devices such as cochlear electrodes incorporating bioresorbable or biodegradable materials, and more particularly to a device which has a first preselected shape suitable for insertion into the body of a patient, and a second shape suitable for providing a specific function, or stimulus, the bioresorbable or biodegredable materials being used to change the device from the second to the first shape.

### Background of the Invention

The invention is described for electrodes used in cochlear implant systems, however, it is equally applicable to other implantable devices. Cochlear implant systems are used to aid patients having a hearing deficiency. More particularly, these systems include a microphone receiving ambient sounds and converting the sounds into corresponding electrical signals, signal processing means of processing the electrical signals and generating cochlea stimulating signals and an electrode assembly for applying the cochlea stimulating signals to the cochlea of the patient. It is known in the art that the cochlea is tonotopically mapped. In other words, the cochlea can be partitioned into regions, with each region being responsive to signals in a particular frequency range. This property of the cochlea is exploited by providing the electrode assembly with an array of electrodes,each electrode being arranged and constructed to deliver a cochlea stimulating signal within a pre-selected frequency range to the appropriate cochlea region. The electrical currents and electric fields from each electrode stimulate the cilia disposed on the modiola of the cochlea. Several electrodes may be active simultaneously. It has been found that in order for these electrodes to be effective, the magnitude of the currents flowing from these electrodes and the intensity of the corresponding electric fields, are a function of the distance between the electrodes and the modiola. If this distance is great, the threshold current magnitude must be larger than if the distance is smaller. Moreover, the current from each electrode may flow in all directions, and the electrical fields corresponding to adjacent electrodes may overlap thereby causing cross-electrode interference. In order to reduce the threshold stimulation amplitude and to eliminate cross-electrode interference, it is advisable to keep the distance between the electrode array and the modiola as small as possible. This is best accomplished by providing the electrode array in the shape which generally follows the shape of the modiola. Of course during insertion, the electrode assembly should be generally straight, because otherwise the insertion procedure is too cumbersome and difficult. Several methods and means of curving the electrode assembly have been reported, however in the opinion of the inventors, none of these prior methods are satisfactory. For example, one electrode assembly is known which includes an electrode carrier provided with a longitudinal element arranged on one side of the carrier and constructed to change its dimension once the assembly is inserted. For example, the longitudinal element could include a hydrogel such as PAA (Polyacrylic acid) which expands after insertion by absorbing water from the cochlear fluid. Alternatively, the longitudinal element could be a bimetallic filament (such as nickel/titanium) which is shaped to allow the electrode carrier to take a straight configuration at room temperature but bends into a preselected shape once it is exposed to body temperature. Another proposed electrode assembly included in a mechanical member arranged to bend the electrode carrier after the carrier has been inserted. All these prior art devices require a structure which is difficult and expensive to manufacture and which in most cases are not expected to perform satisfactorily.

European Patent Application No. 0 002 068 in the name of Hansen et al discloses a cochlear implant device and method in accordance with the precharacterising portions of claims 1 and 12 respectively. More specifically, it discloses an electrode for implantation into a cochlea wherein the curvature of the electrode is controlled by means of a detachable or loosenable connection which, until it is detached, maintains a temporary condition of curvature in electrodes. The detachable connection is disclosed to be thermodetachable, a material that is soluble or swellable in fluid, a detachable connection, or, alternatively, a cord is used to adjust the condition of the curvature of the electrode.

Patent application No. WO 93/06698 in the name of Cochlear et al discloses a method and apparatus by which an electrode can be inserted into the cochlea of a patient and because of a biocompatible material having a property of controlled expansion when exposed to fluid in the cochlea, causes the electrode to assume a preferred contact position within the cochlea.

Patent No. NL 6 709 780 in the name of Seeders provides an intrauterine contraceptive device comprising elements having different water absorbing properties. Once inserted in a uterus differing rates of fluid absorption change the shape of the device so that it is not easily expelled from the uterus.

Patent Application No. WO 83/00011 in the name of Medline AB discloses a plurality of fins associated with the electrode body for positioning the electrode in a second position after insertion. It discloses the use of positioning fins, which, once in contact with body fluids, expand to maintain the position of the inserted body within a body cavity. During the insertion process, the body to be inserted and associated with the fins is disclosed within a bioactive tube, which is removed after insertion of the body.

According to one aspect of the present invention a cochlear implant device comprises:
an elongated member having a plurality of electrodes mounted on said member and having a first configuration selected to allow said member to be inserted into a patient's body and a second configuration for application of therapy, said elongated member being made of a first material, said first material being flexible; and
a second material for biasing said elongated member into said first configuration, said second material being stiffer than said first material, wherein said second material is soluble in body fluids, so that after insertion into said body, said second material dissolves, allowing said member to take said second configuration;
characterized in that said second material is in the form of a sheath enveloping said elongated member and shaped for biasing said elongated member into said first configuration.

Preferably the said elongated member is performed to said second configuration.

Suitably the said elongated member includes a longitudinal body made of an elastic material and a plurality of fins attached to said body, said fins being constructed and arranged for positioning said elongated member to said second configuration after said stiffening member has dissolved.

Conveniently, the said fins are disposed adjacent to said body in said first configuration.

Suitably in the said first configuration said body is substantially straight.

Conveniently said body is provided with a plurality of elastic fins which in said first configuration are disposed adjacent to said body and in said second configuration extend away from said body to engage a cochlear wall.

Preferably said body is formed with a channel, and in said first configuration said fins are disposed in said channel.

Suitably, said body is made of a plastic material with memory and is performed to said first configuration.

Conveniently, said body is made with a plurality of indentations and said stiffening means includes a stiffening material disposed in said indentations.

Preferably said bioresorbable material is selected for the group consisting of polyvinyl alcohol, polylactic acid and polyglycolic acid.

According to a further aspect of the present invention we provide a method of making a therapeutic device said device having a body with a plurality of electrodes on said body and with a first configuration selected for ease of insertion and a second configuration for application of therapy, said method comprising the steps of:
forming said body;
setting said body into said first configuration; and
applying bioresorbable material to said body whereby when said assembly is inserted into a patient, said bioresorbable material dissolves allowing said body to take said second configuration;
characterized in that said bioresorbable material is applied as a sheath enveloping said body.

### BRIEF DESCRIPTION OF THE DRAWINGS

Figure 1 shows a side view of an electrode carrier constructed in accordance with this invention;
Figure 2 shows an electrode assembly constructed in accordance with this invention with the carrier of Figure 1 embedded in a stiffening sheath;
Figure 3 shows a cross-sectional view of the assembly of Figure 2 taken along line 3-3.
Figure 4 shows a cross-sectional view of the scala tympani of a patient with the electrode carrier of Figure 1 after the sheath has been dissolved;
Figure 5 shows a side view of an electrode carrier in accordance with a second alternate embodiment of the invention;
Figure 6 shows the carrier of Figure 5 embedded in a stiffening sheath;
Figure 7 shows a cross-sectional view of the assembly of Figure 6 taken along line 8-8;
Figure 8 shows the carrier of Figure 6 deployed in the scala tympani, after dissolution of the stiffening sheath;
Figure 9 shows a cross-sectional view of the carrier of Figure 8 taken along line 10-10;
Figure 10 shows a somewhat diagrammatic view of the assembly of Figure 6 being inserted in to the scala tympani;
Figure 11 shows a somewhat diagrammatic view of the carrier of Figure 10 deployed in the scala tympani; and
Figure 12 shows the electrode carrier of Figure 10 being withdrawn from the scala tympani.

### Detailed Description of the Invention

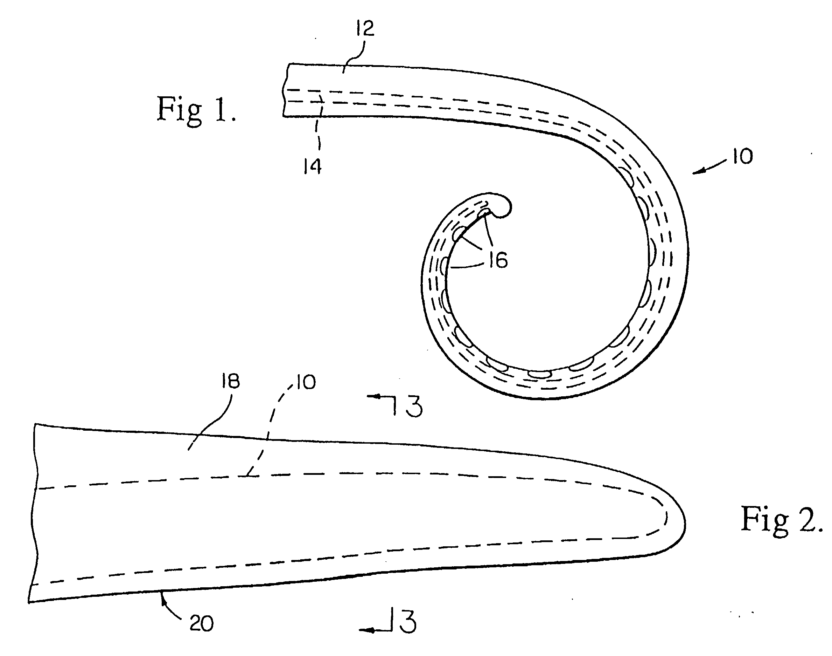

Referring now to Figure 1, an electrode assembly constructed in accordance with this invention includes an electrode carrier 10 formed of an elongated member 12 made of a plastic material such as Silastic ™ MDX 4-4210. Embedded in this member 12 is a cable 14 formed of several insulated wires (not shown), each wire terminating in an electrode 16. The cable 14 is connected to a cochlear stimulation device(not shown) and is used to transmit stimulation pulses from the device to the electrodes 16. As seen in Figure 1, the electrodes 16 are all exposed. The member 12 is elastic, however, it is made from a material which has shape memory and is pre-curved so that its natural configuration is in the shape of a spiral as shown. The curvature of the spiral follows the curvature of the scala tympani of the person, and more particularly, the curvature of the modiola, as shall be described in more detail below.

After its completion, carrier 10 is distorted so that its configuration is substantially straight and is embedded into a sheath 18, as shown in Figure 2, thus forming an electrode assembly 20. Sheath 18 is made of a relatively stiff material so that it is able to retain the carrier 10 in the straight configuration shown. Importantly, sheath 18 is made of a material which dissolves, is bioresorbable or is otherwise biodegradable when immersed in cochlear fluid. For example, sheath 18 may be made of polyvinyl alcohol(PVA), polylactic acid (PLA), polyglycolic acid (PGA)and other similar compounds. Preferably the sheath 18 is made with a smooth outer surface to allow the assembly 20 to be implanted easily. A coating may be applied to this surface to reduce friction. The coating may be made of a time-released antimicrobial material to provide protection against infections during implantation. Sheath 18, while stiffer than carrier 10, must be sufficiently flexible so that it can be bent to conform to the shape of the cochlea.

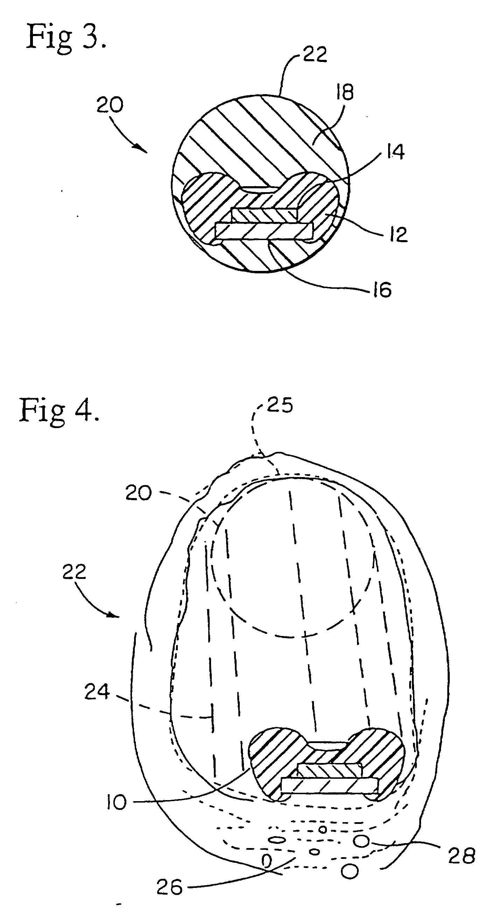

A cross section for the assembly 20 is shown in Figure 3. As can be seen in this Figure, the member 18 is preferably flattened, or in the shape of a figure 8 so that it can bend more easily in a plane normal to the electrode 16.The initial position of electrode assembly 20 immediately after implantation, and the carrier 10 after the sheath 18 is dissolved can be seen in Figure 4. In this Figure, assembly 20 has been implanted into the scala tympani 22 so that it is immersed in the cochlear fluid 24. Initially, because of the stiffness of sheath 18, the assembly maintains a large radius of curvature so that it is disposed adjacent wall 25 of scala tympani 22. However in a relatively short time that can be controlled and is to be determined by the intended insertion procedures the sheath 18 dissolves in the cochlear fluid 24 releasing the carrier 10. As a result the carrier resumes its natural spiral configuration shown in Figure 1. In this configuration, the carrier 10 is disposed adjacent to the modiola 26. Importantly, in this latter configuration the electrodes 16 are facing the modiola 26 so that they are positioned as close as possible to the spiral ganglia 28 thereof. In this manner, the electrodes 16 can generate currents and electric fields with a relatively small intensity without causing cross-channel interference.

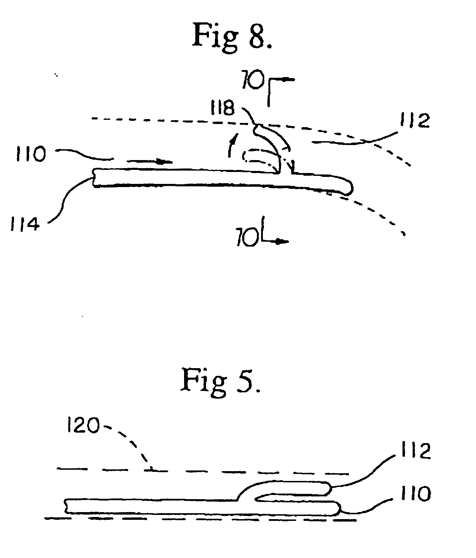

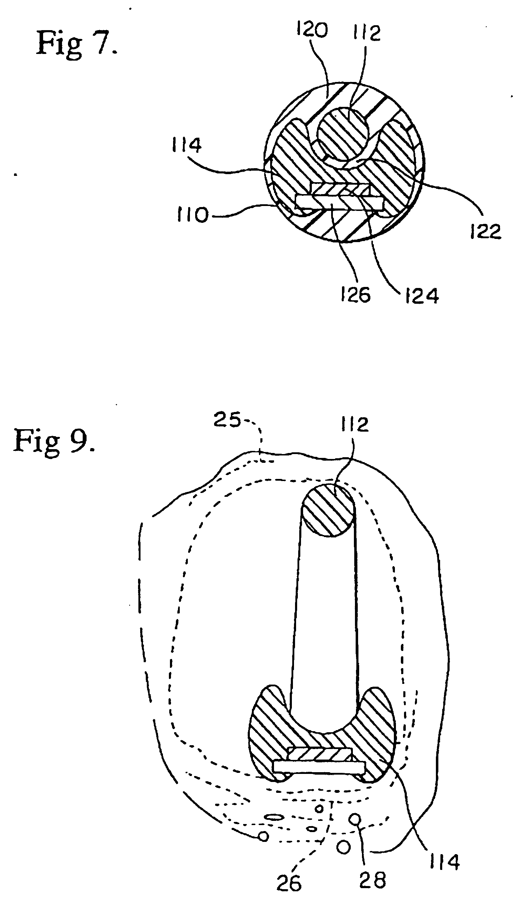

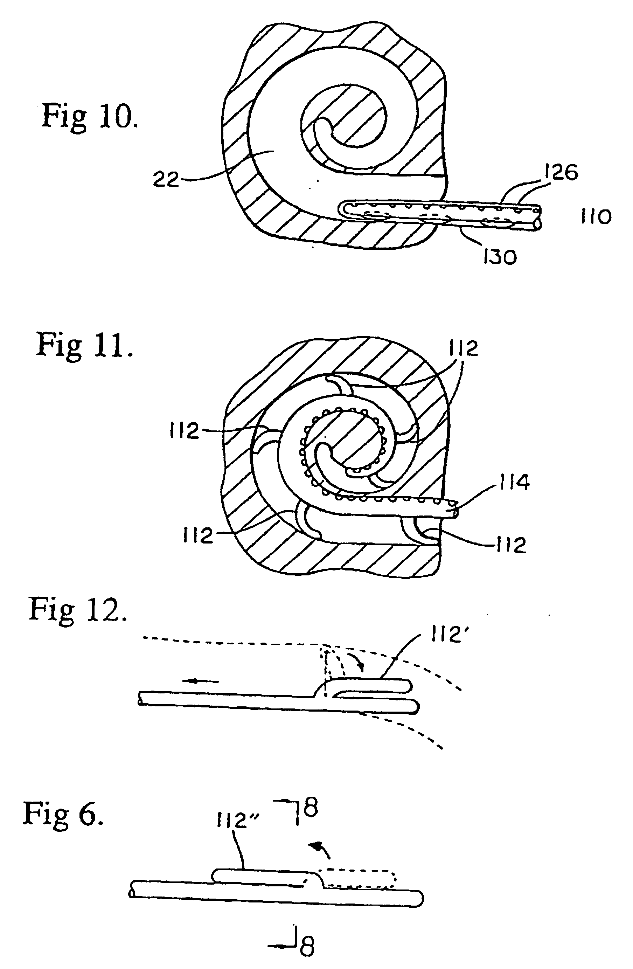

Yet another embodiment of the invention is shown in Figure 5. In this embodiment, carrier 110 is provided with a plurality of longitudinally spaced fins 112 formed on generally tubular body 114. The fins 112 are relatively elastic. In the normal position as shown in Figure 5, these fins 112 extend towards the tip of the carrier. Preferably the free ends 118 of the fins are curved slightly in the longitudinal direction. After the carrier 110 is formed as shown in Figure 5, the fins are forced into a position where they are disposed generally in parallel with and adjacent to the body 114 as shown somewhat diagrammatically in Figure 6. The carrier 110 thus deformed is then encased in a sheath 120 formed of a biodegradable material-like sheath 18. In order to reduce the cross sectional dimensions of the carrier in this second configuration, the body 114 may be formed with a channel. Thus for example, the carrier 110 may include body 114 formed with a channel 122 for housing, in the second configuration, fin 112. As with the previous embodiment, the carrier 110 also includes connecting wires 124 embedded in body 114 and a plurality of electrodes 126. The advantage of this embodiment is that, after the assembly of Figure 7 is implanted, and after the sheath 120 dissolves, the fins 112 are released from channel 122 until their free ends 118 engages wall 25, thereby urging and biasing body 114 toward the modiola 26, as shown in Figures 8 and 9. This whole process is shown in more detail in Figures 10 and 11. The assembly 130 formed of the carrier 110 and sheath 120, as shown in Figure 7, is first inserted into the scala tympani 22. After the assembly 130 is fully inserted, the sheath 120 dissolves leaving the carrier 110 firmly seated with the scala tympani 22 as seen in Figure 11. If it is required to remove the carrier 110 for any reason, as indicated in Figure 12 in the direction A, the fins 112 bend back to the position 112', as shown.

As can be seen in Figure 11, the carrier 110 is firmly seated and positioned in the scala tympani by the interference fit and biasing provided by the fins 112 between the body 114 and the walls of the scala tympani 22. Therefore, the body 114 can, but need no be, made in the spiral shape shown in Figure 1, and need not be made of a shape-retaining material, like carrier 10.

Although the preferred embodiments of the invention have been described in conjunction with a cochlear electrode assembly, it should be understood that its teachings may be applicable for other implanted electrodes, such as the electrodes used in pacemakers. For this latter implementation of the invention, the tines used to secure the distal end of the electrode to an internal cardiac wall, are initially folded and maintained in a closed position by a bioabsorbable sheath in a manner similar to the way the fins 112 are folded in Figure 6. After implantation the sheath dissolves allowing the tines to open and engage the cardiac walls, thereby securing the electrode.

Although the invention has been described with reference to several particular embodiments, it is to be understood that these embodiments are merely illustrative of the application of the principles of the invention. There may be multiple stiffening elements in particular implementations. The stiffening elements may be formed within the structure of the array, rather than on the external surface of it. Accordingly, the embodiments described in particular should be considered exemplary, not limiting, with respect to the following claims.

## Claims

1. A cochlear implant device comprising:
an elongated member (12) having a plurality of electrodes (16) mounted on said member (12) and having a first configuration selected to allow said member (12) to be inserted into a patient's body and a second configuration for application of therapy, said elongated member (12) being made of a first material, said first material being flexible; and
a second material (18) for biasing said elongated member into said first configuration, said second material being stiffer than said first material, wherein said second material is soluble in body fluids, so that after insertion into said body, said second material dissolves, allowing said member (12) to take said second configuration;
**characterized in that** said second material is in the form of a sheath (18) enveloping said elongated member (12) and shaped for biasing said elongated member into said first configuration.

2. The device of claim 1 wherein said elongated member (12) is preformed to said second configuration.

3. The device of claim 1 wherein said elongated member (12) includes a longitudinal body (14) made of an elastic material and a plurality of fins (112) attached to said body (14), said fins (112) being constructed and arranged for positioning said elongated member (12) to said second configuration after said stiffening member (12) has dissolved.

4. The device of claim 3 wherein said fins (112) are disposed adjacent to said body (114) in said first configuration.

5. The device of claim 1 wherein the device is adapted to be a cochlear implant electrode assembly; wherein the elongated member is
an elongated electrode carrier (10) having an elastic body, said body having a first configuration for insertion into a patient's cochlea, and a second configuration in which said carrier (10) is curved to match a surface of said cochlea;
a plurality of electrodes (16) are mounted on said carrier (10); and wherein:
the sheath is a stiffening sheath (18) disposed about said carrier (10) for setting said body (14) into said first configuration, **characterized in that** said stiffening sheath (18) is stiffer than said elastic body (14), said stiffening sheath (18) being made of a bioresorbable material which dissolves an insertion into said cochlea to permit said body (14) to take said second configuration.

6. The device of claim 5 wherein said first configuration said body (14) is substantially straight.

7. The device of claim 5 wherein said body (114) is provided with a plurality of elastic fins (112) which in said first configuration are disposed adjacent to said body (114), and in said second configuration extend away from said body (114) to engage a cochlear wall (25).

8. The device of claim 7 wherein said body (114) is formed with a channel (122), and in said first configuration said fins are disposed in said channel (122).

9. The device of claim 5 wherein said body (114) is made of a plastic material with memory and is preformed to said first configuration.

10. The device of claim 9 wherein said body is made with a plurality of indentations and said stiffening means includes a stiffening material disposed in said indentations.

11. The device of claim 5 wherein said bioresorbable material is selected from the group consisting of polyvinyl alcohol, polylactic acid and polyglycolic acid.

12. A method of making a therapeutic device, said device having a body with a plurality of electrodes (16) on said body and with a first configuration selected for ease of insertion and a second configuration for application of therapy, said method comprising the steps of:
forming said body (14, 114)
setting said body (14, 114) into said first configuration; and
applying bioresorbable material (18, 120) to said body whereby when said assembly is inserted into a patient, said bioresorbable material (18, 120) dissolves allowing said body (14, 114) to take said second configuration,
**characterized in that** said bioresorbable material is applied as a sheath (18, 120) enveloping said body (14, 114).

## Patentansprüche

1. Kochleare Implantat-Anordnung, die Folgendes umfasst:
- ein längliches Bauteil (12) mit einer Vielzahl von Elektroden (16), die auf dem Bauteil (12) befestigt sind und mit einer ersten Ausführung, derart ausgewählt um zu ermöglichen, dass das Bauteil (12) in den Körper eines Patienten eingeführt wird und mit einer zweiten Ausführung zur Anwendung einer Therapie, wobei das längliche Bauteil (12) aus einem ersten Material dargestellt ist, wobei dieses erste Material flexibel ist; und
- ein zweites Material (18) zur Vorspannung des länglichen Bauteiles in die erste Ausführung, wobei das zweite Material steifer als das erste Material ist, wobei das zweite Material in Körperflüssigkeiten löslich ist, so das nach der Einführung in den Körper sich das zweite Material auflöst, wodurch ermöglicht wird, dass das Bauteil (12) die zweite Ausführung annimmt;
- **dadurch gekennzeichnet, dass** das zweite Material die Form einer Hülle (18) aufweist, die das längliche Bauteil (12) einhüllt und zur Vorspannung des länglichen Bauteiles in die erst Ausführung geformt ist.

2. Anordnung nach Anspruch 1, worin das längliche Bauteil (12) in die zweite Ausführung vorgeformt ist.

3. Anordnung nach Anspruch 1, worin das längliche Bauteil (12) einen aus einem elastischen Material hergestellten langgestreckten Körper (14) aufweist und eine Vielzahl von Lamellen (112) beinhaltet, die an den Körper (14) angebracht sind, wobei die Lamellen (112) derart aufgebaut und angeordnet sind, dass sie das längliche Bauteil (12) in die zweite Ausführung positionieren nachdem das Versteifungsbauteil (12) aufgelöst ist.

4. Anordnung nach Anspruch 3, worin die Lamellen (112) in der ersten Ausführung nahe dem Körper (114) positioniert sind.

5. Anordnung nach Anspruch 1, worin die Anordnung angepasst ist, um einen kochlearen Implantat-Elektrodenaufbau darzustellen, worin das längliche Bauteil dient als:
- ein verlängerter Elektrodenträger (10) mit einem elastischen Körper, wobei der Körper eine erste Ausführung zur Einführung in die Kochlea eines Patienten aufweist und eine zweite Ausführung in welcher der Träger (10) gekrümmt ist um mit einer Oberfläche der Kochlea zusammenzupassen;
- eine Vielzahl von Elektroden (16) ist auf dem Träger (10) befestigt; und worin:
- die Hülle eine Verstärkungshülle (18) ist, die über dem Träger (10) zum Einbringen des Körpers (14) in die erste Ausführung eingestellt ist, **dadurch gekennzeichnet, dass** die Versteifungshülle (18) steifer ist als der elastische Körper (14), wobei die Versteifungshülle (18) aus einem bioresorbierbaren Material hergestellt ist, welches sich bei der Einführung in die Kochlea auflöst, womit ermöglicht wird, dass der Körper (14) die zweite Ausführung annimmt.

6. Anordnung nach Anspruch 5, worin die erste Ausführung des Körpers (14) im Wesentlichen gerade ist.

7. Anordnung nach Anspruch 5, worin der Körper (114) mit einer Vielzahl von elastischen Lamellen (112) ausgestattet ist, welche in der ersten Ausführung benachbart zu dem Körper (114) angeordnet sind und in der zweiten Ausführung sich von dem Körper (114) weg erstrecken um in eine kochleare Wand (25) einzugreifen.

8. Anordnung nach Anspruch 7, worin der Körper (114) mit einem Kanal (122) ausgebildet ist und in der ersten Ausführung die Lamellen in dem Kanal (122) angeordnet sind.

9. Anordnung nach Anspruch 5, worin der Körper (114) aus einem Kunststoffmaterial mit Memory-Effekt hergestellt ist und in der ersten Ausführung vorgeformt ist.

10. Anordnung nach Anspruch 9, worin der Körper mit einer Vielzahl von Vertiefungen hergestellt ist und Versteifungsmittel ein Versteifungsmaterial beinhalten, welches in den Vertiefungen angeordnet ist.

11. Anordnung nach Anspruch 5, worin das bioresorbierbare Material ausgewählt ist aus der Gruppe von Polyvinyl-Alkohol, Polylaktiksäure und Polyglykolsäure.

12. Verfahren zur Herstellung einer therapeutischen Anordnung, wobei die Anordnung einen Körper mit einer Vielzahl von Elektroden (16) auf dem Körper aufweist und mit einer ersten Ausgestaltung, ausgewählt zur leicht gängigen Einführung und einer zweiten Ausgestaltung zur Anwendung in einer Therapie, wobei das Verfahren folgende Schritte aufweist:
- Ausbildung des Körpers (14,114);
- Einstellen des Körpers (14,114) in die erste Ausführung; und
- Anwendung eines bioresorbierbaren Materials (18,120) in dem Körper, womit, falls der Aufbau in einen Patienten eingeführt wird, sich das bioresorbierbare Material (18,120) auflöst, womit für den Körper (14,114) die Einnahme der zweiten Ausführung ermöglicht wird;
**dadurch gekennzeichnet, dass** das bioresorbierbare Material als eine Hülle (18,120) angewandt wird, welche den Körper (14,114) einhüllt.

## Revendications

1. Dispositif d'implant cochléaire comprenant :
un élément allongé (12) doté d'une pluralité d'électrodes (16) montées sur ledit élément (12) et ayant une première configuration choisie pour permettre audit élément (12) d'être inséré dans l'organisme d'un patient et une seconde configuration pour une application thérapeutique, ledit élément allongé (12) étant fait d'un premier matériau, ledit premier matériau étant flexible ; et
un second matériau (18) visant à conformer ledit élément allongé dans ladite première configuration, ledit second matériau étant plus rigide que ledit premier matériau, dans lequel ledit second matériau est soluble dans les fluides organiques, de sorte qu'après insertion dans ledit organisme, ledit second matériau se dissout, permettant audit élément (12) de prendre ladite seconde configuration ;
**caractérisé en ce que** ledit second matériau est sous la forme d'un étui (18) enveloppant ledit élément allongé (12) et façonné pour conformer ledit élément allongé dans ladite première configuration.

2. Dispositif selon la revendication 1 dans lequel ledit élément allongé (12) est préformé dans ladite seconde configuration.

3. Dispositif selon la revendication 1 dans lequel ledit élément allongé (12) comprend un corps longitudinal (14) fait d'un matériau élastique et d'une pluralité d'ailettes (112) fixées audit corps (14), lesdites ailettes (112) étant construites et agencées de façon à placer ledit élément allongé (12) dans ladite seconde configuration après que ledit élément raidisseur (12) se soit dissous.

4. Dispositif selon la revendication 3 dans lequel lesdites ailettes (112) sont disposées adjacentes audit corps (114) dans ladite première configuration.

5. Dispositif selon la revendication 1 dans lequel le dispositif est adapté pour être un ensemble d'électrodes pour implant cochléaire ; dans lequel l'élément allongé est
un porteur d'électrodes allongé (10) ayant un corps élastique, ledit corps ayant une première configuration pour une insertion dans la cochlée d'un patient, et une seconde configuration dans laquelle ledit porteur (10) est incurvé pour s'adapter à une surface de ladite cochlée ;
une pluralité d'électrodes (16) sont montées sur ledit porteur (10) ; et dans lequel :
l'étui est un étui raidisseur (18) disposé autour dudit porteur (10) pour placer ledit corps (14) dans ladite première configuration, **caractérisé en ce que** ledit étui raidisseur (18) est plus rigide que ledit corps élastique (14), ledit étui raidisseur (18) étant fait d'un matériau biorésorbable qui dissout une insertion dans ladite cochlée pour permettre audit corps (14) de prendre la seconde configuration.

6. Dispositif selon la revendication 5 dans lequel ladite première configuration dudit corps (14) est essentiellement droite.

7. Dispositif selon la revendication 5 dans lequel ledit corps (114) est doté d'une pluralité d'ailettes élastiques (112) qui, dans ladite première configuration, sont disposées adjacentes audit corps (114), et, dans ladite seconde configuration, s'éloignent dudit corps (114) pour entrer en prise avec une paroi cochléaire (25).

8. Dispositif selon la revendication 7 dans lequel ledit corps (114) est formé d'un canal (122), et dans ladite première configuration lesdites ailettes sont disposées dans ledit canal (122).

9. Dispositif selon la revendication 5 dans lequel ledit corps (114) est fait d'un matériau plastique doté de mémoire et est préformé en ladite première configuration.

10. Dispositif selon la revendication 9 dans lequel ledit corps est fait d'une pluralité d'indentations et ledit moyen raidisseur comprend un matériau raidisseur disposé dans lesdites indentations.

11. Dispositif selon la revendication 5 dans lequel ledit matériau biorésorbable est choisi dans le groupe constitué par l'alcool polyvinylique, l'acide polylactique et l'acide polyglycolique.

12. Procédé de fabrication d'un dispositif thérapeutique, ledit dispositif ayant un corps doté d'une pluralité d'électrodes (16) sur ledit corps et avec une première configuration choisie pour une facilité d'insertion et une seconde configuration pour une application thérapeutique, ledit procédé comprenant les étapes consistant à :
former ledit corps (14, 114)
placer ledit corps (14, 114) dans ladite première configuration ; et
appliquer un matériau biorésorbable (18, 120) audit corps moyennant quoi lorsque ledit ensemble est inséré dans l'organisme d'un patient, ledit matériau biorésorbable (18, 120) se dissout laissant ledit corps (14, 114) prendre ladite seconde configuration,
**caractérisé en ce que** ledit matériau biorésorbable est appliqué comme un étui (18, 120) enveloppant ledit corps (14, 114).
